(19) Europäisches Patentamt
European Patent Office
Office européen des brevets

(11) Veröffentlichungsnummer: **0 254 869**
**A2**

(12) # EUROPÄISCHE PATENTANMELDUNG

(21) Anmeldenummer: **87109036.1**

(22) Anmeldetag: **24.06.87**

(51) Int. Cl.4: **A61K 31/215**, A61K 31/455, //A61K31/07,A61K31/44,A61K3-1/355,A61K31/12,A61K31/59,A-61K31/57

(30) Priorität: **30.06.86 DE 3621861**

(43) Veröffentlichungstag der Anmeldung: **03.02.88 Patentblatt 88/05**

(84) Benannte Vertragsstaaten: **AT BE CH ES FR GB GR IT LI NL SE**

(71) Anmelder: **Ilg, Laszlo, Dr.**
**Zermatter Strasse 29**
**D-2800 Bremen 41(DE)**

Anmelder: **Gligora, Mario, Dr.**
**A. Kovacica 22**
**Y-5100 Rijeka(YU)**

(72) Erfinder: **Ilg, Laszlo, Dr.**
**Zermatter Strasse 29**
**D-2800 Bremen 41(DE)**
Erfinder: **Gligora, Mario, Dr.**
**A. Kovacica 22**
**Y-5100 Rijeka(YU)**

(74) Vertreter: **Hansen, Bernd, Dr.rer.nat. et al**
**Hoffmann, Eitle & Partner Patentanwälte**
**Arabellastrasse 4**
**D-8000 München 81(DE)**

(54) **Verwendung von Aryloxycarbonsäure-Derivaten gegen dermatologische Erkrankungen.**

(57) Es wird die Verwendung von Aryloxycarbonsäure-Derivaten zur Herstellung eines Arzneimittels gegen dermatologische Erkrankungen, insbesondere zur Bekämpfung von Ulcus cruris, Striae distense atrophicae, Cellulitis, Kollagenosen (Lupus erythematosus, Sklerodermie, Dermatomyositis, etc.), REM-Syndrom (Reticular erythematos-Syndrom), Alopecia mucinosa, Necrobiosis lipoidica diabeticorum, Paniculitis nodosa, Folliculitis decalvans, Psoriasis vulgaris, Elastosis senilis, Störungen und Erkrankungen der elastischen Fasern, mesenchymale Tumore, Kollagen-und Gefässerkrankungen der Niere, Leber und Lunge, Muskelerkrankungen, Mesenchymaltumore, Verbrennungen, Cicatrix, Lichen sclerosus und atrophicans, Anetodermiae (Atrophiae cutis), immunologische Erkrankungen
(erworbenes-Immunschwäche-Syndrom [AIDS]) und als Antagonist gegen Corticosteroide,beschrieben.

EP 0 254 869 A2

Xerox Copy Centre

## Verwendung von Aryloxycarbonsäure-Derivaten gegen dermatologische Erkrankungen

Die Erfindung betrifft die Verwendung von Aryloxycarbonsäure-Derivaten zur Herstellung eines Arzneimittels gegen dermatologische Erkrankungen, insbesondere zur Bekämpfung von Striae distense atrophicae, Ulcus cruris, Cellulitis, REM-Syndrom (Reticular erythematos-Syndrom), Alopecia mucinosa, Necrobiosis lipoidica diabeticorum, Paniculitis nodosa, Folliculitis decalvans, Psoriasis vulgaris, mesenchymale Tumore, Cicatrix, immunologische Erkrankungen (erworbenes Immunschwäche-Syndrom [AIDS]) und als Antagonist gegen Corticosteroide.

In DE-OS 36 06 041 wird die Verwendung von Aryloxycarbonsäure-Derivaten zur Bekämpfung von Effluvium oleosum capilicium, nämlich Haarausfall wegen Überfettung des Haaransatzes, beschrieben. Dabei wird direkt auf die Lipozyte bzw. auf Matrix und Bulbus des Haares medikamentös eingewirkt, ohne dass dabei schädliche Nebenwirkungen verursacht werden.

In den Lipozyten läuft die Fettsynthese ab. Gruppen von Lipozyten bilden "Knötchen" (Globuli). Die Knötchen sind mit Collagen und retikulären Fasern umgeben. Dieses Kapillarsystem dient für den Übergang des Fettes von den Zellen in das Blut. Zahlreiche Lipogene und lipobiotische Faktoren haben Einfluss auf die Lipozyten der Subcutis.

Die Lipozyten weisen in der Hauptsache Triglyceride auf, und zwar vor allem in Verbindung mit Palmitinsäure, Stearinsäure und ungesättigten Fettsäuren. Die Fettverhältnisse in den Lipozyten sind ähnlich denen im Blutplasma. Durch topische Anwendung von Lipidsenkern aus der Gruppe der Aryloxycarbonsäure-Derivate konnte eine andauernde Verbesserung der Haarsituation ohne das Auftreten von unerwünschten Nebeneffekten erzielt werden.

Ähnliche Störungen im Fett-und Talkhaushalt liegen bei einer Reihe von dematologischen Erkrankungen vor, wie insbesondere bei Cellulitis, Paniculitis nodosa, Psoriasis vulgaris, Alopecia mucinosa, REM-Syndrom (Reticular erythematos-Syndrom), Folliculitis decalvans, Necrobiosis lipoidica diabeticorum, mesenchymale Tumore, Cicatrix, ausserdem können die erfindungsgemässen Verwendungen als Antagonisten gegen Corticosteroide eingesetzt werden.

Im Falle von Striae distense atrophicae stellt man bei histologischen Versuchen eine Verminderung der elastischen Fasern fest. Die Epidermis ist atrophisch. Die epidermalen Annexe fehlen. Die Hautzeichnung sowie Haare fehlen in den Striae, ausserdem befinden sich die Striae unter dem Niveau der gesunden Haut. Es ist bekannt, dass Collagen und Leukozyten sich aus den gleichen mesenchymalen Zellen in der embrionalen Phase entwickeln. Es besteht somit ein Bedarf nach einem Präparat, das auf die Lypozyten einwirkt und gleichzeitig zu einer Verminderung von überschüssigem Fett führt.

Aufgabe der vorliegenden Erfindung ist es, die Verwendung eines Arzneimittels vorzuschlagen, das sich in einfacher Weise und besser als die bisher verwendeten Medikamente zur Bekämpfung von dermatologischen Erkrankungen, insbesondere der vorstehend aufgeführten Symptome sowie Ulcus cruris und anderer eignet, indem es direkt auf die Störungen des umgebenden Fettgewebes wirkt. Von besonderer Bedeutung ist dabei, dass es nicht zum Auftreten von Nebeneffekten kommt.

Die vorstehende Aufgabe wird gemäss der Erfindung durch die Verwendung von Aryloxycarbonsäure-Derivaten gelöst, die sich zur Bekämpfung dermatologischer Erkrankungen, insbesondere von Ulcus cruris, Striae distense atrophicae, Cellulitis, Collagenosen (Lupus erythematosus, Sklerodermie, Dermatomyositis, etc.), REM-Syndrom (Reticular erythematos-Syndrom), Alopecia mucinosa, Necrobiosis lipoidica diabeticorum, Paniculitis nodosa, Folliculitis decalvans, Psoriasis vulgaris, Elastosis senilis, Störungen und Erkrankungen der elastischen Fasern, mesenchymale Tumore, Kollagen-und Gefässerkrankungen der Niere, Leber und Lunge, Muskelerkrankungen, Mesenchymaltumore, Verbrennungen, Cicatrix, Lichen sclerosus und atrophicans, Anetodermiae (Atrophiae cutis), immunologische Erkrankungen (erworbenes-Immunschwäche-Syndrom [AIDS]) und als Antagonist gegen Corticosteroide eignen.

Die erfindungsgemäss verwendeten Arzneimittel eignen sich bei allen Veränderungen, die verursacht werden durch Krankheiten des Mesenchyms, unabhängig vom Organ und Ätiologie, wie z.B. Ulceration im Nasen-Rachen-Raum, Mund-, Speiseröhre-und Magengeschwüre. Unter den Medikamenten mit lipidsenkender Wirkung aus der Gruppe der Aryloxycarbonsäure-Derivate, insbesondere der Aryloxyessigsäure-Derivate, die sich zur Bekämpfung von Effluvium oleosum capilitium eignen, sind Verbindungen der allgemeinen Formel

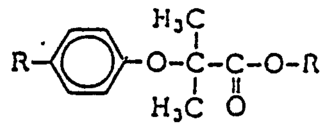

zu verstehen. Die Substituenten R können dabei die nachfolgend angegebenen Bedeutungen haben. Die entsprechenden Derivate sind im Handel als bekannte Arzneimittel erhältlich.

| | | |
|---|---|---|
| Cl- | $-C_2H_5$ | >Clofibrat< Atheropront®, Regelan® Amotril®, Atromid® |
| Cl- | 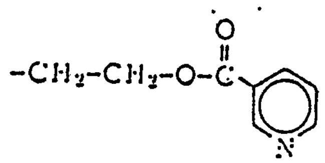 | >Etofibrat< Lipo-Merz® |
| Cl- | 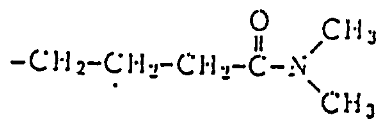 | >Clofibrid< Lipenan® |
| 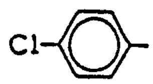 | $-CH_3$ | >Methylclofenapat< |
| 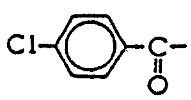 | $-C_3H_7-i$ | >Procetofen< Lipantyl® |
| 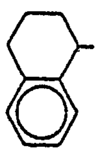 | -H | >Nafenopin< Melipan® |

Im weiteren sind auch noch zwei Ester der Clofibrinsäure mit symmetrischen bifunktionellen Alkoholen zu nennen. Beim Simfibrat ist die Alkoholkomponente 1,3-Propandiol, während im Tiafibrat die Molekülkomponente von Tiadenol enthalten ist:

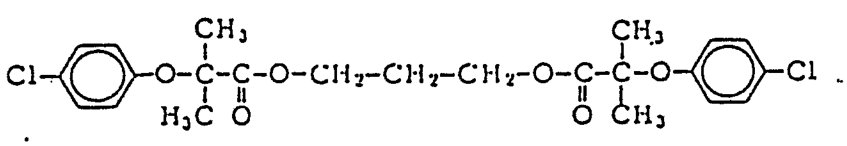

>Simfibrat< Diclofibrat Cholesolvin®

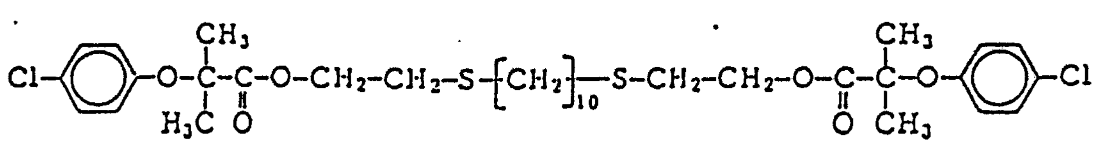

>Tiafibrat<

Weitere gemäss der Erfindung bevorzugt verwendete Verbindungen sind Fenofibrat (2-Methyl-2-[4-(4-chlorbenzoyl)-phenoxy]-propionsäure-isopropylester) sowie Acetofilinfibrat.

Aus der Verwendung der vorstehend genannten Lipidsenker zur Behandlung von Hyperlipidämie ist bekannt, dass diese den Cholesterin-Spiegel sowie insbesondere den Gehalt an Triglyceriden im Blut merklich absenken. Ausserdem wird eine Reduzierung des Gehaltes an freien Fettsäuren beobachtet.

Die vorstehend genannten lipidsenkenden Substanzen werden als alkoholische Lösung oder als Suspension in Alkohol, insbesondere Ethanol oder Isopropylalkohol, sowie Spiritus dilutus in 0,5 bis 5 %iger, vorzugsweise 0,5 bis 3 %iger Lösung topisch auf der Kopfhaut angewendet. Dabei ist es bevorzugt, die Lösung täglich ein-bis dreimal, besonders bevorzugt zweimal, aufzutragen, wobei dies geeigneterweise unter Verwendung eines Wattebausches erfolgen kann.

Ausserdem ist es bevorzugt, die Wirkstoffe als 2-bis 4-%iges, vorzugsweise 2%iges Gel (z.B. Glyceringel) oder als 3-bis 5-%ige Salbe (Vaselinum album) zu formuliern.

Um die Wirkung der den Lipidsenker enthalteden Lösung bzw. Suspension, Gel oder Salbe noch weiter zu optimieren, ist. es bevorzugt, dass dieser Lösung oder Suspension Vitamine zugegeben werden, deren positiver Effekt auf das Haarwachstum bzw. auf den Fettstoffwechsel bekannt ist Hierbei sind insbesondere zu nennen: Vitamin A, Vitamin B, besonders bevorzugt Vitamin $B_6$, das eine juckreizhemmende Wirkung hat, Vitamin D, Vitamin E und/oder Vitamin K. Die Zugabemenge von Vitaminen zu der erfindungsgemäss verwendeten Lösung bzw. Suspension richtet sich nach dem zu behandelnden Krankheitsbild. Die nachfolgend angegebenen Vitaminmengen stellen die bevorzugten Bereiche dar:

Vitamin A 200-2000 I. E. auf 100 ml am meisten bevorzugt 500-1000 I.E.
Vitamin B, 0,01-0,1 %
insbes. $B_6$ am meisten bevorzugt Q,25 %
Vitamin D 100-500 I.E. auf 100 ml
besonders bevorzugt 200 I.E.
Vitamin E 0,005-0,1 %,
besonders bevorzugt 0,01 %

Darüber hinaus ist es zur Verbesserung des erfindungsgemäss verwendeten Mittels vorteilhaft, Dexpanthenol (D-(+)-2,4-Dihydroxy-3,3-dimethyl-N-(3-hydroxypropyl)-buttersäureamid bzw. Panthenol zuzugeben. Mittels der genannten Substanzen wird eine verstärkte Tiefenwirkung des Präparates erzielt. Die vorstehend genannten Substanzen werden vorzugsweise in einer Konzentration von 0,0005 bis 0,01 % zugegeben, besonders bevorzugt in einer Menge von 0,001 %.

Im weiteren ist es bevorzugt, der erfindungsgemäss verwendeten Lösung, Suspension, dem Gel oder der Salbe ein Antibiotikum zuzugeben. Als Antibiotika eignen sich dafür insbesondere Neomycin, Gentamycin, Erytromycin. Die Antibiotika werden dabei in einer Menge von 0,05 bis 2 %, besonders bevorzugt von 0,5 bis 1 %, der erfindungsgemäss verwendeten Lösung bzw. Suspension, dem Gel oder der Salbe zugegeben.

Darüber hinaus kann der erfindungsgemäss verwendeten Lösung bzw. Suspension, dem Gel oder der Salbe Cortison in geigneter Menge, z.B. 0,1 bis 2 %, zugegeben werden.

Die erfindungsgemäss verwendeten Arzneimittel, die Derivate der Aryloxycarbonsäure enthalten, können topisch, per os, parenteral, subkutan, intrarektal verabreicht werden. Besonders bevorzugt ist die topische Anwendung derselben.

Im folgenden wird eine gemäss der Erfindung zu verwendende Lösung einer Arzneimittelformulierung angegeben:

Clofibrat 1 %
Dexpanthenol 0,01%
Vitamin A 500 I.E.
Vitamin $B_6$ 0,25 %
Vitamin D 200 I.E.
Vitamin E 0,01 %
sterilisiertes Wasser Rest.

Die folgenden Versuche beschreiben die erfindungsgemässe Verwendung:

1. Behandlung von Striae distense

Die Striae distensae cutis sind parallel verlaufende, manchmäl wellige Streifen, ca. 15 mm lang, zunächst rötlich oder blaurötlich, später weissrötlich oder gelblich im Farbton, selten hyperpigmentiert. Die leicht eingesunkene Haut ist verdünnt, fein, quergefältet, gelegentlich auch hernienartig vorgewölbt. Bei Spannung zeigen sie vermehrten Glanz. Der häufigste Sitz der Striae sind die Lumbosacralregion, Oberschenkel, Trochanter und Suprapatellargegend sowie Bauch und Brust. Am häufigsten kommen sie in der Pubertät vor (bei Mädchen 70 %, bei Jungen 40%), in der Schwangerschaft zu 90 % im letzten Trimenon. Bei der weissen Rasse treten sie häufiger auf als bei der schwarzen. Im histologischen Bild ist eine atrophische Epidermis sichtbar, das Kollagen vollständig homogenisiert. Die elastischen Fasern sind fragmentiert, bilden dreieckförmige Herde mit subpapillar gelegener Basis und der Spitze in der Subcutis. Am Rande dieser Herde sind die elastischen Fasern normal, oft angehäuft. Aus der Histogenese der Haut wissen wir, dass sich die Fibroblasten aus dem Mesenchym entwickeln. Die Fibroblasten produzieren die Vorstufen der Kollagen-und elastischen Fasern.

Die bisherige Therapie mit Vitaminen und Massagen war weitgehend erfolglos. Zur Behandlung von Striae distensae wurde daher eine Therapie mit einem 2%igen Glycerin-Clofibrat-Gel angewendet. Dabei sollte auf die krankhaften Zustände des Mesenchyms direkt eingewirkt werden.

Die Therapie wurde täglich durchgeführt, ausser sonntags, mit bis zu 20 Sitzungen von jeweils zwischen 5 und 20 Minuten.

Es wurden folgende Gruppen behandelt:

1. 12 Patientinnen mit Striae am Bauch. Sie wurden mit dem vorstehend genannten Gel unter Anwendung von Ultraschall behandelt (5 Minuten; 0,8 bis 1,0 W/$cm^2$).

2. 16 Patientinnen mit Striae an den Oberschenkeln. Sie wurden ebenfalls mit dem oben genannten Gel unter Anwendung von Ultraschall behandelt (10 bis 20 Minuten; 0,6 bis 0,8 W/$cm^2$).

3. Eine Patientin mit Striae an den Nates. Sie wurde mit dem oben genannten Gel zusammen mit Ultraschall behandelt ( 8 bis 10 Minuten; 0,8 bis 1,0 w/$cm^2$).

Ergebnisse:

Bei der Verwendung von Glycerin-Clofibrat-Gel ohne Ultrabeschallung kam es zu einer Verminderung der Striae; diese wurden enger. Bei zusätzlicher Ultrabeschallung erfolgte eine schnellere Wirkung. Die Striae wurden enger; manche bis zu 3 cm langen Striae verschwanden gänzlich. Die Haut in der Umgebung wurde elastischer.

Nach der Behandlung konnte festgestellt werden, dass die Striae auf das gleiche Niveau zurückkommen wie die Haut. Es kommt zu einer normalen Hautzeichnung sowie zu selten auftretenden Flaumhaaren. Die Farbe der Striae ändert sich; diese sind nicht mehr weiss, sondern haben einen gelben Schimmer. Es ist bekannt, dass Collagen der gesunden Haut einen gelben Schimmer verleiht. Dies bedeutet, dass man bereits makroskopisch Kollagen nachweisen kann. Histologische Untersuchungen ergeben folgendes:

Unter der weiterhin atrophischen Epidermis beobachtet man die Ausbildung einer wellenförmigen Grenze und kann andeutungsweise Papillen feststellen. Neues Kollagen ist subepidermal sichtbar. Die elastischen Fasern sind weiterhin diskontinuierlich, d.h. zerfetzt, aber vermehrt. Das Kollagen im tiefen Corium ist ebenfalls verbessert.

2. <u>Behandlung von Ulcus cruris</u>

Für die folgenden Untersuchungen wurden Präparate eingesetzt, die als Wirkstoffe Clofibrat und Fenofibrat enthielten. Die Anwendung erfolgte örtlich beim Ulcus cruris verschiedener Ätiologie, wie postthrombotischer Ulcus, posttraumatischer Ulcus, trophischer Ulcus, arterieller Ulcus und Necrosis lipoidica diabeticorum. Die Konzentration der Wirkstoffe betrug 0,5 bis 5%, insbesondere 4% bei Behandlungsbeginn und später 2 bis 3%. Das Medikament wurde entweder als Gel oder in Salbenform verabreicht.

Das Patientenalter lag zwischen dem 47. und 81. Lebensjahr. Bei Behandlungsbeginn war die Krankheit zwischen 3 Monate und 3 Jahre alt.

Die Grösse des Ulcus lag bei 10-Pfennigstück-gross bis handflächengross, mit meist scharf geschnittenen Rändern verschiedener Tiefe. Die Form war sehr variabel (rundlich, langgestreckt, unregelmässig bizarr). Der Ulcusboden war mit schmierigem nekrotischem Belag bedeckt.

Die besten Ergebnisse wurden erreicht mit einer Kombinationsbehandlung, bei der am Anfang ein 4%iges Gel bzw. eine entsprechende Salbe, und später ein 2%iges Gel (oder Salbe) aufgetragen wurde. Bei den niedrigen Konzentrationen wurde nur eine langsame Wundheilung beobachtet. Bei Verwendung von 5%igem Gel kam es zu einer hypertrophischen Granulation im Ulcusbodenbereich ohne Epithelisierungstendenz. Unter gleichzeitiger Verwendung von Cortisonsalben verschwand das hypertrophische Granulationsgewebe.

Am Anfang der Behandlung wurde das Gel bzw. die Salbe dreimal täglich auf die Wunde appliziert und später, nachdem sich der Ulcusboden angehoben und sich der Umgebung angeglichen hatte, zweimal täglich. Die Bodengranulation war meistens mit Randepithelisation begleitet, selten mit inselartier Epithelisierung im Ulcus.

Bei allen 16 behandelten Patienten kam es zu einer Epithelisierung. Bei der Hälfte der Patienten (8) kam es innerhalb von 2 1/2 Monaten zu einer vollkommenen Ausheilung. Bei den übrigen wurde eine deutliche Heilungstendenz beobachtet. Zusammenfassend lässt sich sagen, dass bei allen Patienten eine positive Wirkung des Medikamentes zu verzeichnen war.

Bei sämtlichen Patienten wurden in zweiwöchentlichen Abständen bakteriologische Untersuchungen durchgeführt. Bei Behandlungsanfang konnte nur eine Bakterienart festgestellt werden. Im späteren Behandlungsverlauf wurden bis zu drei verschiedene Bakterienarten beobachtet. Die Wundheilung wurde dadurch nicht gestört.

Bei 4 Patienten wurde vor und nach der Therapie eine histologische Untersuchung durchgeführt, wobei das Gewebe vom Ulcusrand entnommen wurde:

-Histologischer Befund vor der Therapie mit Derivaten der Aryloxycarbonsäure:

Über dem Ulcusboden findet man Fibrin mit vereinzelten nekrotischen Zellresten. Unter dem Ulcus zeigt sich unspezifisches Granulationsgewebe ohne Kollagen.

-Histologischer Befund nach der Therapie mit Derivaten der Aryloxycarbonsäure:

Die Epidermis ist erhöht mit zahlreichen Zellen, die sich in der Mitose befinden. Im Corium findet sich Granulationsgewebe mit neugebildeten Kollagenfasern wie auch neugebildeten Kapillargefässen mit etwas vergrösserten Endothelzellen und hyperchromatischen Zellkernen.

Bei sämtlichen Patienten führte die Therapie des Ulcus cruris mit Derivaten der Aryloxycarbonsäure zu guten Ergebnissen. Bei keinem Patienten wurde eine Empfindlichkeit auf das lokal angewendete Medikament festgestellt.

3. Behandlung von Cellulitis (mit Ultraschall)

An 20 Patientinnen wurde mit Hilfe einer 1%igen Clofibrat-Lösung eine Cellulitis-Behandlung durchgeführt, wobei ein gutes Ergebnis erhalten wurde.

4. Behandlung von Psoriase

Es ist bekannt, dass im Falle von Psoriase der Lipidgehalt in der Schuppen um 35 % höher ist als in der normalen Haut.

In diesem Fall wurde eine 3-bis 5%ige Salbe verwendet, über welche dann ein Verband angelegt wurde. Es zeigt sich, dass durch die Einwirkung von Glycerin-Clofibrat-Gel die Schuppen schneller verschwanden als bei Anwendung eines Placebos. Bereits nach 6 Tagen waren mit Hilfe des Wirkstoffes die ersten Erfolge zu verzeichnen. Ohne weitere Behandlung kam es dann zu einem Rezidiv. Daraus ist abzuleiten, dass die Verwendung der Salbe besonders geeignet ist in Kombination mit einem Korticosteroid oder einem vergleichbaren Präparat.

5. Behandlung von Folliculitis decalvans

Die sehr selten vorkommende Krankheit wurde an einer Patientin mit Hilfe von 2%iger Clofibrat-Lösung behandelt. Bereits nach 20 Behandlungstagen kam es zu einem erneuten Haarwuchs im alopetischen Areal, während es bei den bisherigen Behandlungsmethoden immer zu einem endgültigen Haarausfall kam.

6. Behandlung von Alopecia mucinosa und REM-Syndrom

Die Behandlung mit 1 %iger Clofibrat-Lösung zeigte sich im Falle von Alopecia mucinosa erfolgreich: Das Haar war neu gewachsen, die ödematos infiltrierten entzündlichen, geröteten Herde bildeten sich zurück. Die Schuppung hörte auf.

7. Behandlung von Necrobiosis lipoidica diabeticorum

Hier erfolgte eine Behandlung mit 1%iger Clofibrat-Lösung bzw. Acetofilinfibrat-Lösung. Die Therapie war erfolgeich. Das Erythem und Ödem bildeten sich zurück.

8. Behandlung von mesemchymalen Tumoren (multiplen Lipomen) (mit Ultraschall)

Es wurde eine Patientin mit multiplen Lipomen mit 3%igem Clofibrat-Glycerin-Gel und zusätzlicher Ultrabeschallung behandelt. Nach 20 Behandlungen wurde festgestellt, dass die Lipome weicher und weniger auffällig waren.

**Ansprüche**

1. Verwendung von Aryloxycarbonsäure-Derivaten zur Herstellung eines Arzneimittels gegen dermatologische Erkrankungen.

2. Verwendung nach Anspruch 1, zur Herstellaung eines Arzneimittels gegen Ulcus cruris.

3. Verwendung von Aryloxycarbonsäure-Derivaten zur Herstellung eines Arzneimittels gegen Striae distense atrophicae, Cellulitis, Collagenosen (Lupus erythematosus, Sklerodermie, Dermatomyositis), REM-Syndrom (Reticular erythematos-Syndrom), Alopecia mucinosa, Necrobiosis lipoidica diabeticorum, Paniculitis nodosa, Folliculitis decalvans, Psoriasis vulgaris, mesenchymale Tumore, Collagen-und Gefässerkrankungen der Niere, Leber und Lunge, Muskelerkrankungen, Mesenchymaltumore, Verbrennungen, Cicatrix, immunologische Erkrankungen (erworbenes Immunschwäche-Syndrom [AIDS]) und als Antagonist gegen Corticosteroide.

4. Verwendung nach Anspruch 1, wobei Clofibrat eingesetzt wird.

5. Verwendung nach Anspruch 1, wobei Fenofibrat eingesetzt wird.

6. Verwendung nach Anspruch 1, wobei Etofibrat (Etilfibrat) eingesetzt wird.

7. Verwendung nach einem oder mehreren der Ansprüche 1 bis 6, wobei eine topische Anwendung erfolgt.

8. Verwendung nach einem oder mehreren der Ansprüche 1 bis 7, wobei das Aryloxycarbonsäure-Derivat in Lösung, Suspension, als Gel oder Salbe vorliegt.

9. Verwendung nach einem oder mehreren der Ansprüche 1 bis 8, wobei im weiteren Vitamin A, B, insbesondere $B_6$, D, E und/oder K zugesetzt wird.

10. Verwendung nach einem oder mehreren der Ansprüche 1 bis 9, wobei im weiteren Cortison zugegeben wird.